(19) Europäisches Patentamt / European Patent Office / Office européen des brevets

(11) **EP 1 791 792 B2**

(12) **NOUVEAU FASCICULE DE BREVET EUROPEEN**
Après la procédure d'opposition

(45) Date de publication et mention de la décision concernant l'opposition:
**25.01.2023 Bulletin 2023/04**

(45) Mention de la délivrance du brevet:
**31.05.2017 Bulletin 2017/22**

(21) Numéro de dépôt: **05805575.7**

(22) Date de dépôt: **06.09.2005**

(51) Classification Internationale des Brevets (IPC):
*C02F 1/52* (2006.01)     *A61L 11/00* (2006.01)

(52) Classification Coopérative des Brevets (CPC):
**C02F 1/529; A61L 11/00; C02F 1/5245**

(86) Numéro de dépôt international:
**PCT/FR2005/002215**

(87) Numéro de publication internationale:
**WO 2006/030102 (23.03.2006 Gazette 2006/12)**

(54) **UTILISATION DE CHAUX PARTIELLEMENT PRE-HYDRATEE DANS LA SEPARATION D'UN MELANGE MATIERES SOLIDES/LIQUIDE, PROCEDE DE TRAITEMENT DES BOUES ET BOUES PURIFIEES OBTENUES SELON CE PROCEDE**

VERWENDUNG VON TEILWEISE VOR-HYDRATIERTEM KALZIUMOXID ZUM TRENNEN EINER MISCHUNG AUS FESTEN/FLÜSSIGEN STOFFEN, VERFAHREN ZUR BEHANDLUNG DES DURCH BESAGTES VERFAHREN ENTSTANDENEN SCHLAMMS

USE OF PARTLY PRE-HYDRATED LIME FOR SEPARATING A MIXTURE OF SOLID/LIQUID MATTERS, METHOD FOR TREATING SLUDGE AND PURIFIED SLUDGE OBTAINED BY SAID METHOD

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **15.09.2004 FR 0409767**

(43) Date de publication de la demande:
**06.06.2007 Bulletin 2007/23**

(60) Demande divisionnaire:
**16150530.0**

(73) Titulaires:
• **Gombart, Marc**
  **45160 Ardon (FR)**
• **Carmeuse Chaux**
  **62320 Bois Bernard (FR)**

(72) Inventeurs:
• **GOMBART, Marc**
  **F-45160 Ardon (FR)**

• **TILQUIN, Jean-Yves**
  **B-4530 Villers-Le-Bouillet (BE)**
• **BARTIAUX, Stéphane**
  **B-7500 Tournai (BE)**

(74) Mandataire: **AWA Benelux**
  **Parc d'affaires Zénobe Gramme - Bât. K**
  **Square des Conduites d'Eau 1-2**
  **4020 Liège (BE)**

(56) Documents cités:
  **WO-A1-98/02391     FR-A- 2 841 895**
  **JP-A- 60 054 799**

• **"19- Traitement des Boues" In: DEGRÉMONT: "Memento technique de l'eau, Neuvième édition", 1989 pages 949-957,**

EP 1 791 792 B2

## Description

### Domaine de l'invention

La présente invention a trait à une utilisation de chaux partiellement pré-hydratée, qui contient du CaO réagissant avec $H_2O$ après un certain temps de latence, c'est-à-dire une chaux vive à réactivité retardée (en abrégé :"CVRR"), dans le domaine de la séparation de matières solides d'un mélange comprenant lesdites matières solides et un liquide (i. e. séparation liquide/solide, désignée ci-après par commodité "séparation matières solides/liquide" ou "séparation solides/liquide"), et en particulier dans le domaine de la séparation des matières solides d'une suspension dans un liquide. Elle concerne principalement un procédé de traitement d'une boue, dans lequel on concentre, décontamine, sèche et recueille les matières solides au moyen de ladite chaux partiellement pré-hydratée à réactivité retardée. Elle concerne enfin, en tant que produits industriels, (a) la boue purifiée (désignée ci-après "matériau solide issu d'une boue") obtenue selon ce procédé, d'une part, et (b) la combinaison d'une CVRR avec au moins un sel de $Fe^{3+}$ et/ou de $Al^{3+}$, qui est utile pour mettre en oeuvre ce procédé, d'autre part.

### Art antérieur

On sait que différentes techniques de séparation solides/liquide ont été développées pour concentrer les matières solides contenues dans un mélange matières solides/liquide quand au moins une portion desdites matières solides est dispersée dans le liquide dudit mélange, notamment lorsqu'elles sont sous la forme de suspensions de particules, comme cela est le cas pour les boues.

Lesdits mélanges concentrés ainsi obtenus, notamment les suspensions concentrées, doivent souvent subir des traitements ultérieurs avant de pouvoir être manipulés, stockés ou réutilisés. Ces traitements mettent en oeuvre des réactifs dont le rôle est par exemple de modifier les propriétés chimiques, de décontaminer (i. e. d'hygiéniser ou dépolluer) ou encore de modifier les propriétés physiques du mélange matières solides/liquide concentré ou de la suspension concentrée, telles que la rhéologique ou la plasticité.

On sait que la concentration finale en matières solides d'un mélange solides/liquide ou d'une suspension est un facteur important qu'il convient d'optimiser afin de favoriser la mise en oeuvre des étapes de traitement, de manutention et de stockage. Dans cette optique, il a été proposé d'améliorer les performances des méthodes de séparation matières solides/liquide, par exemple :

- d'une part, au moyen d'un traitement chimique des mélanges matières solides/liquide ou des suspensions conduisant à des phénomènes de précipitation et de floculation,

- d'autre part, en optimisant l'outil lui-même, le terme "outil" désignant ici les modalités et les dispositifs de séparation solides/liquide [en particulier les centrifugeuses et appareillages de filtration tels que les filtres-presse (notamment les filtres-presse à plateaux ou à tubes) et les filtres à bande (notamment les filtres à bande pressée ou sous vide)].

Il est également connu que la chaux peut être utilisée pour améliorer les performances des méthodes de séparation matières solides/liquide. Elle est alors ajoutée à la suspension avant la séparation matières solides/liquide (la personne du métier parle dans ce cas de "pré-chaulage", i. e. opération de chaulage avant filtration, centrifugation ou décantation) ou après ladite séparation (la personne du métier parle alors de "post-chaulage", i. e. opération de chaulage après filtration, centrifugation ou décantation).

### Pré-chaulage

Dans le cadre du pré-chaulage, il se trouve que la chaux vive ne convient pas quand le mélange solides/liquide ou suspension contient un liquide réagissant avec elle, tel que l'eau. En effet, la réaction violente entre la chaux vive et l'eau (i) conduit à des mélanges non homogènes, et (ii) perturbe la séparation matières solides/liquide. On préfère donc introduire, dans ledit mélange ou ladite suspension à concentrer, de la chaux éteinte $Ca(OH)_2$, soit sous forme de poudre, soit sous forme de suspension aqueuse. Les particules de $Ca(OH)_2$, ainsi ajoutées ou formées par réaction de la chaux avec l'eau du mélange matières solides/liquide ou de la suspension, présentent une taille typiquement inférieure à 10 $\mu$m ; ces particules sont trop petites pour pouvoir augmenter la porosité du résidu ou gâteau de séparation et favoriser ainsi la dessiccation et la décontamination du gâteau de séparation matières solides/liquide.

Même si l'utilisation de chaux vive ajoutée sous la forme de particules plus grosses est susceptible de favoriser la centrifugation des boues en augmentant la porosité du gâteau de filtration ou centrifugation [comme décrit dans l'article publié dans Wat. Sci. Tech., 1993, Vol.28 (No.1), pp 223-231] le problème de la réaction violente entre la chaux et l'eau du mélange matières solides/liquide ou de la suspension, qui n'est pas résolu, reste rédhibitoire.

On sait de l'article de S. DENNEUX-MUSTIN et al., Wat. Res., 2001, vol.35 (No. 12), pp 3018-3024, que l'utilisation de chaux associée à $FeCl_3$ favorise la déshydratation mécanique des boues. Cet effet est également observé lorsque la chaux est ajoutée seule à une boue contenant déjà du fer comme décrit dans WO 99/10288 A.

On sait aussi que, lors de l'utilisation de floculants polymères organiques, l'ajout de chaux engendre un pH élevé qui provoque, dans la plupart des cas, leur inactivation ou destruction par hydrolyse alcaline. Le brevet délivré EP 1154958 B propose de contourner cette difficulté en contrôlant ou retardant l'augmentation du pH ; la solution préconisée à cet effet comprend l'addition à la boue à concentrer d'un floculant organique et d'une chaux particulière dans le cadre du pré-chaulage, ladite chaux étant choisie parmi l'ensemble constitué (selon le libellé de la revendication 1 dudit brevet) par :

- la chaux vive,

- la chaux éteinte sous forme pulvérulente,

- la chaux éteinte en suspension dans de l'eau ayant une taille de particules $d_{50} \geq 50\ \mu m$,

- la chaux surcuite,

- la chaux vive contenant un additif fluide présentant un pouvoir d'agglomération des particules fines,

- la chaux vive contenant un agent retardateur d'hydratation,

- la chaux éteinte présentant des particules élémentaires agglomérées,

- la chaux éteinte additionnée d'un agent ralentisseur d'activité [i. e., selon le langage utilisé par EP 1154958 B au § *[0019]*, un produit qui ralentit la dissolution de $Ca(OH)_2$ dans l'eau],

- la chaux éteinte présentant une teneur en matière sèche supérieure à 20 % en poids, et

- la chaux "défillérisée" [i. e., une chaux dans laquelle les particules fines ont été enlevées, en particulier celles ayant une taille inférieure ou égale à 200 $\mu m$].

La description de EP 1154958 B signale en outre la possibilité de faire appel à une CVRR pour l'opération de pré-chaulage (voir à cet effet la dernière phrase du § *[0026]* de ce brevet) afin de ralentir la réaction d'extinction. Cependant ce brevet ne décrit ni ne suggère la caractéristique de la présente invention, à savoir que ladite CVRR intervient dans l'agglomération des matières solides lors de l'opération de pré-chaulage; puis dans l'opération de post-chaulage pour la dessiccation et la décontamination des matières solides contenues dans les boues.

Post-chaulage

Par ailleurs, l'utilisation de chaux vive, au cours du post-chaulage des mélanges matières solides/liquide concentrés ou des suspensions concentrées contenant chacun de l'eau, présente certes de nombreux avantages. On sait en particulier de WO 02/32818 A que l'augmentation du pH et de la température associée à la réaction de la chaux vive avec l'eau résiduelle contenue dans les gâteaux humides de séparation assure leur hygiénisation. En effet, outre le fait de libérer de la chaleur, il se trouve que la réaction (1) entre l'eau et la chaux vive :

$$(1) \qquad CaO + H_2O \rightarrow Ca(OH)_2$$

consomme de l'eau et permet d'accroître le niveau de siccité du gâteau de séparation, comme indiqué dans US 4279279 A, la chaux vive CaO étant en effet capable de capter environ 32% de son poids en eau.

Cependant, comme dans le cas du pré-chaulage, il est également difficile d'obtenir en post-chaulage un mélange homogène à cause de la violence de la réaction (1) précitée. De plus le post-chaulage nécessite une étape supplémentaire, à savoir le mélange dudit mélange matières solides/liquide concentré, ou de ladite suspension concentrée, avec la chaux vive. Cette étape de mélange présente l'inconvénient d'augmenter le coût et la complexité des modalités opératoires, d'une part, et de déstructurer ledit mélange matières solides/liquide concentré ou ladite suspension concentrée, que constitue le gâteau de séparation, par les contraintes mécaniques qu'elle génère, d'autre part.

L'inconvénient lié à la multiplication des étapes de traitement du gâteau de séparation se rencontre dans US 5679262 A, qui décrit une technique de post-chaulage comportant deux étapes. Cette technique permet certes de diminuer de 40 % la quantité de chaux vive nécessaire pour le traitement d'une boue, mais a le désavantage de rendre plus complexe les modalités de mise en oeuvre dudit traitement.

CVRR

On connaît par ailleurs des chaux vives partiellement pré-hydratées à réactivité retardée (CVRR). Ces produits sont constitués de CaO et de $Ca(OH)_2$. Ils se présentent sous la forme d'un mélange de particules de chaux vive CaO et de particules de chaux éteinte $Ca(OH)_2$, agencées de telle façon que, en présence d'eau, la réaction (1) précitée soit retardée. Dans l'état actuel des connaissances, on a tout lieu de présumer que la CVRR se présente sous la forme de particules de chaux vive CaO, constituant un noyau, et de particules de chaux éteinte $Ca(OH)_2$, constituant une couche ou pellicule temporairement protectrice enrobant le noyau de CaO. Même s'il s'agit là d'une théorie qui ne lie pas le Demandeur, la représentation "noyau de CaO enrobé dans une membrane ou couche de $Ca(OH)_2$ temporairement protectrice" est bien pratique pour appréhender le mécanisme du retard de la réaction (1). A ce sujet, la demande française publiée FR 2841895 A propose en particulier une CVRR utile, en tant qu'agent liant pour compacter une charge minérale (telle que l'argile, la terre, le ciment, les boues et leurs mélanges), dans la fabrication d'articles façonnés. Selon l'enseignement de FR 2841895 A, ladite CVRR intervient, dans la fabrication desdits articles façonnés, sous la forme d'une poudre ou, grâce à l'ajout d'un plastifiant (désigné "superplastifiant" par la personne du métier), sous la forme d'une suspension aqueuse fluide et, le cas échéant, concentrée.

Compactage

Il convient de ne pas confondre le compactage (notamment selon JP 60/054799 A ou FR 2841895 A précité) d'un déchet, tel qu'une boue, avec l'opération de pré-chaulage ou celle de post-chaulage. Le compactage d'un déchet contenant de l'eau consiste, par addition de CaO, à enrober ledit déchet dans une matrice sèche de $Ca(OH)_2$. Le compactage d'un déchet ne contenant pas d'eau comprend le mélange dudit déchet avec CaO puis l'addition de $H_2O$ pour obtenir la même matrice sèche. Le compactage, réalisé en particulier dans un moule à parois rendues antiadhérentes, conduit à l'obtention de produits façonnés.

**But de l'invention**

Il existe un besoin en ce qui concerne le problème technique de la concentration, de la séparation et du séchage, au moyen de chaux vive ou éteinte, des produits solides contenus dans les mélanges matières solides/liquide ou dans les suspensions dont le liquide (l'eau ou un solvant organique) est susceptible de réagir avec la chaux vive. L'idéal serait d'avoir les avantages des opérations dites de pré-chaulage et de post-chaulage sans leurs inconvénients précités, d'une part, et de disposer d'un mélange homogène chaux/matières solides à recueillir (i) dans ledit mélange matières solides/liquide ou la suspension à traiter puis (ii) dans le résidu de séparation sans manipulation supplémentaire contraignante et sans réaction violente du CaO avec $H_2O$ ou ledit liquide organique dudit mélange ou de ladite suspension, ladite réaction violente ayant le désavantage d'être susceptible de générer de la poussière et de produire des agglomérés de chaux empêchant l'obtention de l'homogénéité souhaitée, d'autre part.

Aussi, on se propose de fournir une nouvelle solution technique au problème du traitement des mélanges matières solides/liquide ou des suspensions comportant un liquide capable de réagir avec CaO, en vue de concentrer, décontaminer (i.e. "hygiéniser" ou dépolluer), dessécher et recueillir les matières solides sèches contenues dans lesdits mélanges ou suspensions, notamment les suspensions aqueuses, d'une part.

On se propose également d'appliquer cette nouvelle solution technique plus particulièrement au traitement des boues, d'autre part.

On se propose en outre de fournir un procédé de traitement des dites boues afin de les concentrer, décontaminer, sécher et recueillir, par exemple en vue de leur épandage sans risque de pollution pour l'environnement, notamment pour améliorer ou amender les sols cultivables.

On propose enfin de fournir, en tant que produits industriels (a) les boues purifiées obtenues selon ce procédé, et (b) une combinaison d'une CVRR avec au moins un sel de fer et/ou d'aluminium.

**Objet de l'invention**

Ce but a été atteint grâce à la mise en oeuvre d'une chaux réactive particulière, à savoir une chaux vive partiellement pré-hydratée à réactivité retardée (CVRR), susceptible d'être obtenue selon l'enseignement du document FR 2 841 895 A précité, et qui intervient dans l'opération de pré-chaulage par le $Ca(OH)_2$ protecteur qu'elle contient, puis dans l'opération de post-chaulage par le CaO qu'elle contient.

Dans ce qui suit, le terme "suspension" englobe, par commodité, toute suspension proprement dite (i. e. présence de particules solides dispersées dans le liquide) et tout mélange matières solides/liquide dans lequel au moins une portion des matières liquides (si infime soit elle) est susceptible d'être dispersée (notamment par agitation) dans le liquide. En effet, même si dans ledit mélange matières solides/liquide les particules solides sont substantiellement rassemblées, il suffit de l'agiter pour obtenir la dispersion des particules solides qu'il contient; en variante, on peut soutirer une portion du surnageant (ou dans certains cas du sousnageant) et mettre sous agitation la composition solides/liquide restante pour avoir ladite dispersion. Si nécessaire, ledit surnageant (ou sousnageant) ainsi soutiré, qui peut être trouble, peut à son tour être retraité et concentré.

On préconise une utilisation de chaux vive dans la concentration, séparation et dessiccation de matières solides susceptibles d'être dispersées (notamment sous agitation, la dispersion pouvant être au moins partielle) dans un liquide, ladite utilisation faisant appel à une chaux vive partiellement pré-hydratée à réactivité retardée (CVRR), qui est constituée de deux composants essentiels, CaO et $Ca(OH)_2$, agencés de telle façon que, en présence d'eau, la réaction (1), entre la chaux vive CaO et l'eau :

$$(1) \qquad CaO + H_2O \rightarrow Ca(OH)_2$$

soit temporairement retardée par la chaux éteinte $Ca(OH)_2$, la chaux éteinte de la CVRR servant à la floculation ou concentration des matières solides avant séparation, et la chaux vive de ladite CVRR servant à la dessiccation desdites matières solides pendant ou (mieux) après séparation.

Selon l'invention, on préconise un nouveau procédé pour la concentration, la séparation, la dessiccation et la décontamination des matières solides d'une boue au moyen de chaux en vue de séparer ses matières solides, ledit procédé étant réalisé selon la revendication 1.

Le procédé selon l'invention permet d'obtenir en tant que produit industriel un matériau solide issu d'une boue, ledit matériau ayant été purifié au moyen d'une CVRR constituée de 40 à 98 % en poids de CaO et de 60 à 2 % en poids de $Ca(OH)_2$, de préférence constituée de 80 à 92 % en poids de CaO et de 20 à 8 % en poids de $Ca(OH)_2$, et mieux constituée de 85 à 90 % en poids de CaO et de 15 à 10 % en poids de $Ca(OH)_2$, ayant une granulométrie moyenne telle que 20 $\mu$m $\leq d_{50} \leq$ 200 $\mu$m et étant utilisée (i) sous forme de poudre ou (ii) sous forme de suspension aqueuse à une concentration de supérieure ou égale à 10 % en poids.

On préconise pour le traitement d'une boue une combinaison de

(a) une CVRR, constituée de 40 à 98 % en poids de CaO et de 60 à 2 % en poids de $Ca(OH)_2$, de préférence constituée de 80 à 92 % en poids de CaO et de 20 à 8 % en poids de $Ca(OH)_2$, et mieux constituée de 85 à 90 % en poids de CaO et de 15 à 10 % en poids de $Ca(OH)_2$, ayant une granulométrie moyenne telle que 20 $\mu$m $\leq d_{50} \leq$ 700 $\mu$m et étant utilisée (i) sous forme de poudre ou (ii) sous forme de suspension aqueuse à une concentration supérieure ou égale à 10 % en poids,

avec

(b) au moins un sel métallique, notamment un sel de $Fe^{3+}$ et/ou un sel de $Al^{3+}$, de préférence $FeCl_3$, $Al_2(SO_4)_3$, et/ou le chlorure basique d'aluminium,

dans laquelle la CVRR et le sel métallique sont conditionnés séparément, sous la forme de deux produits distincts, ou ensemble, sous la forme d'un seul produit résultant de leur mélange,

ladite combinaison étant notamment utile pour (i) la séparation matières solides/eau d'une boue industrielle, d'une boue urbaine, d'une boue d'eaux usées, d'une boue biologique, d'une boue agricole telle que le lisier, ou d'une boue de dragage, et (ii) la décontamination, le séchage et la rectification de la formulation des matières solides résultant de ladite séparation.

## Brève description du dessin

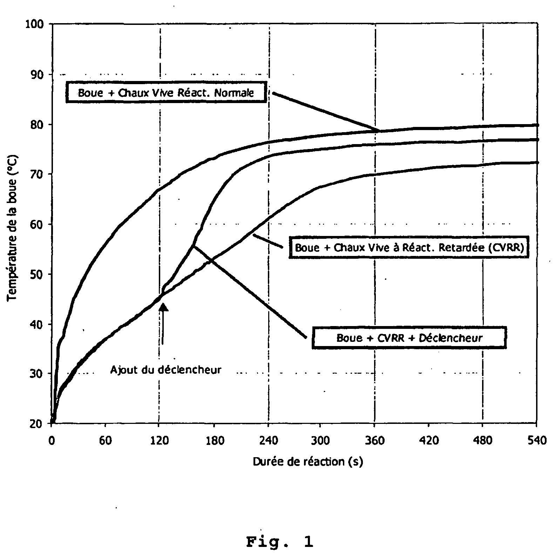

Dans le dessin annexé, la figure unique (Fig. 1) permet de comparer les réactivités de plusieurs chaux dans le système température de la boue (en °C) en ordonnées en fonction de la durée de réaction (en secondes) en abscisses.

## Description détaillée de l'invention

Dans l'expression "chaux vive à réactivité retardée", on entend que le CaO contenu dans la CVRR présente, en contact avec de l'eau, une réactivité retardée de l'ordre de quelques minutes à 2 heures voire plus selon FR 2841895 A, par rapport à celle de la chaux vive non pré-hydratée. Le retard de réactivité augmente avec la teneur en $Ca(OH)_2$ de la CVRR. En outre il est fonction de la concentration et de la nature des matières constitutives de la suspension que l'on veut traiter.

Le liquide de la suspension, qui convient selon l'invention, est constitué par ou contient de l'eau, d'une part, ou est constitué par un ou plusieurs solvants pouvant réagir avec le CaO de la CVRR, d'autre part. Par commodité, les suspensions dont il est question ci-après, notamment dans les exemples, sont des suspensions aqueuses.

Quand il s'agit de granulométrie, par

$$d_x \leq y \text{ (en } \mu m)$$

on entend ici le fait que x % en poids des particules concernées ont une taille inférieure ou égale à y $\mu$m.

Le taux de siccité (TC) correspond au pourcentage de matière sèche dans une suspension. Il est calculé ici par la relation :

$$TC = 100 \cdot \frac{MSO}{MSU}$$

où MSO est la masse des matières solides contenues dans la suspension, et MSU est la masse totale de la suspension.

Le gain de siccité est également exprimé en pourcentage et correspond à la différence entre le TC de la suspension avant séparation et le TC de la suspension après séparation :

$$\text{gain de siccité} = TC_{avant} - TC_{après}$$

Selon l'invention, on ajoute en amont de l'étape de séparation des solides du liquide une CVRR, notamment telle que décrite dans le document antérieur FR 2841895 A précité.

Quand la CVRR est ajoutée à la suspension lors de l'étape de pré-chaulage, chaque noyau de chaux vive ne réagit pas avec l'eau contenue dans la suspension, eu égard au temps de latence de la réaction (1) du fait de sa couche protectrice de Ca(OH)$_2$. Cette circonstance permet d'obtenir facilement un mélange homogène entre la chaux et la suspension et d'éviter de perturber le procédé de séparation solides/liquide. En outre, de manière surprenante, le contact entre la couche de Ca(OH)$_2$ et la suspension génère un pH suffisamment élevé pour favoriser la séparation solides/liquide, le cas échéant en association avec des sels métalliques. La granulométrie de cette chaux permet également d'accroître la porosité des gâteaux et favorise les performances des systèmes de séparation mécanique comme les centrifugeuses, les filtres-presse et les filtres à bande.

Après séparation, la couche de Ca(OH)$_2$ se résorbe et libère le noyau de chaux vive qu'elle enrobe. Ce noyau de CaO va alors réagir avec l'eau résiduelle présente dans le gâteau ou résidu de séparation. Cette réaction produira de la chaleur, engendrera un pH élevé et assurera ainsi l'hygiénisation. En outre, cette réaction augmentera encore le taux de siccité du gâteau ou résidu de séparation. Le traitement est alors plus performant et plus économique que celui décrit dans les solutions techniques antérieurement connues, car au moment de la réaction, la chaux est déjà bien dispersée de façon homogène dans ledit gâteau ou résidu de séparation, et que l'on évite une étape coûteuse et longue de mélange. En bref, l'invention présente enfin l'avantage de n'utiliser qu'une seule chaux particulière, la CVRR, pour mettre en oeuvre de façon combinée et successive les opérations de pré-chaulage puis post-chaulage.

Comme indiqué plus haut, la CVRR est constituée de 40 à 98 % en poids de CaO et de 60 à 2 % en poids de Ca(OH)$_2$. De préférence la CVRR sera constituée de 80 à 92 % en poids de CaO et de 20 à 8 % en poids de Ca(OH)$_2$, et mieux de 85 à 90 % en poids de CaO et de 15 à 10 % en poids de Ca(OH)$_2$.

Ladite CVRR est selon l'invention utilisée (i) sous forme de poudre ou (ii) sous forme de suspension aqueuse à une concentration supérieure ou égale à 10 % en poids. Quand elle intervient sous forme de suspension aqueuse, ladite CVRR sera de façon avantageuse à une concentration pouvant être supérieure ou égale à 50 % en poids, et mieux à une concentration de 60 à 90 % en poids. En bref utilisée sous forme de suspension aqueuse, ladite CVRR sera à une concentration de 5 à 90 % en poids, les concentrations élevées (par exemple de l'ordre de 75 à 90 % en poids) pouvant être obtenues par addition d'un superplastifiant connu dans l'art.

La solution technique de l'invention s'applique tout particulièrement au traitement des boues, notamment aux :

- boues industrielles,

- boues de dragage,

- boues agricoles telles que les lisiers,

- boues biologiques (pouvant contenir des microorganismes susceptibles d'être pathogènes pour l'environnement),

- boues issues du traitement des eaux usées, et

- boues urbaines.

La CVRR est d'abord mélangée avec la suspension à concentrer et à traiter à laquelle on a éventuellement déjà ajouté la solution de sels métalliques, idéalement du chlorure ferrique, du sulfate d'aluminium et/ou du chlorure basique d'aluminium [on rappelle que le chlorure basique d'aluminium, également dénommé "oxychlorure d'aluminium", est un produit ayant pour formule : $Al_2(OH)_3Cl.2H_2O$ selon le *MERCK INDEX 13ème édition, 2001*]. Le mélange peut être réalisé par n'importe quelle technique, mais de préférence dans un malaxeur. Durant la phase de mélange, la granulométrie de la CVRR n'évolue pas ou peu. Elle sera donc ajustée pour optimiser le fonctionnement de l'outil et les performances du procédé de dessiccation. En ce qui concerne la granulométrie de la CVRR, il s'agissait de trouver un compromis optimisé entre la nécessité d'avoir des particules de petite taille pour assurer une bonne répartition de la chaux et la nécessité d'avoir des particules de taille suffisante pour accroître la porosité du gâteau ou du résidu et favoriser la séparation solides/liquide.

La solution technique préconisée ici est que ladite CVRR utilisée a une granulométrie inférieure à 5 mm. On recommande, pour disposer d'une bonne porosité au niveau du gâteau ou résidu de séparation, une CVRR ayant de préférence une granulométrie moyenne comprise entre 20 $\mu$m et 1 mm. En pratique, on éliminera les particules ayant une taille supérieure à 1 mm pour éviter les phénomènes de décantation notamment pour les suspensions présentant des viscosités faibles. Sauf cas particulier, le $d_{50}$ ne sera pas choisi inférieur à 20 $\mu$m afin de prévenir la diminution de la porosité des gâteaux ou résidus de séparation.

La granulométrie moyenne de la CVRR, que l'on recommande tout particulièrement selon l'invention, est telle que 20 $\mu$m $\leq d_{50} \leq$ 700 $\mu$m. Si cela est souhaitable, il est même possible de tamiser la CVRR pour écarter les particules ayant une taille supérieure ou égale à 700 $\mu$m, puis celles ayant une taille inférieure ou égale à 20 $\mu$m.

La chaux sera dosée en fonction de la teneur en matières sèches de l'émulsion à traiter, des caractéristiques rhéologiques attendues pour le produit concentré final, des contraintes de pH ou de température imposées notamment par l'hygiénisation dudit produit final. En pratique, on utilisera au moins 1 partie en poids de CVRR pour 100 parties en poids sec de matières solides contenues dans l'émulsion à traiter.

La chaux selon l'invention sera avantageusement utilisée, notamment pour le traitement d'une boue industrielle, urbaine ou biologique, selon une quantité de CVRR, exprimée en équivalents de CaO, de 3 à 100 parties en poids pour 100 parties en poids sec de matières solides, et mieux de 20 à 100 parties en poids pour 100 parties en poids sec de matières solides, contenues dans la boue à traiter, ladite CVRR intervenant

(i) sous la forme d'une poudre, ou

(ii) sous la forme d'une suspension aqueuse à une concentration supérieure ou égale à 10 % en poids, de préférence à une concentration supérieure ou égale à 50 % en poids, et mieux (en association avec un superplastifiant) à une concentration de 75 à 90 % en poids.

A l'étape ($\alpha$) du procédé de l'invention, on met en contact et sous agitation, pendant 0,4 à 30 minutes, en particulier pendant une durée inférieure ou égale à 10 minutes, et notamment pendant 2 à 3 minutes, la CVRR avec la boue à traiter, le $Ca(OH)_2$ des particules de ladite CVRR servant à la concentration ou floculation desdites matières solides.

Une durée inférieure ou égale à 10 minutes est surtout recommandée afin d'assurer l'intégrité des noyaux de chaux vive de la CVRR pendant l'étape ($\alpha$). Grâce à sa granulométrie, ladite CVRR agit comme minéral sur la porosité du gâteau ou résidu de séparation et favorise l'obtention de gains de siccité.

A l'étape ($\beta$) de ce procédé, on sépare par filtration, centrifugation ou décantation le matériau humide obtenu, qui comprend les matières solides de la suspension initiale, les particules de la CVRR et de l'eau. Cette opération est réalisable au moyen d'un dispositif approprié, par exemple une centrifugeuse, un filtre-presse ou un filtre à bande, voire encore une cuve de décantation.

A l'étape ($\gamma$) du procédé de l'invention, on peut laisser réagir le CaO de la CVRR avec l'eau du gâteau ou résidu de séparation. Cependant, afin de favoriser la séparation solides/liquide et/ou éviter des temps d'attente trop longs, on prévoit selon l'invention la possibilité de provoquer, dans la suspension concentrée, la réaction entre CaO et l'eau. Il existe également un intérêt de pouvoir localiser la réaction à un endroit où l'on peut maîtriser et valoriser le dégagement de chaleur et éventuellement de gaz, comme dans le cas du $NH_3$ libéré lors du traitement des suspensions contenant des sels d'ammonium comme les lisiers. Ainsi la réaction de l'étape ($\gamma$) peut avantageusement être initiée :

◦ par voie thermique, notamment par apport de vapeur sèche ;

◦ par voie sonique, notamment par un champ d'ultrasons ; ou

◦ par voie chimique, notamment avec une substance choisie parmi l'ensemble constitué par les acides minéraux, les acides organiques, les sels alcalins, les sels alcalino-terreux, les polyols et leurs mélanges.

Le déclenchement peut être effectué par activation thermique, sonique ou en ajoutant un déclencheur chimique. L'objectif de ce déclenchement est de détruire la couche d'hydroxyde de calcium et de libérer les noyaux de chaux vive. Les déclencheurs chimiques sont des substances réagissant avec l'hydroxyde de calcium ou pouvant modifier sa solubilité. Ils seront préférentiellement choisis parmi l'ensemble comprenant les acides inorganiques (notamment HCl ou $HNO_3$), les acides organiques, les sels alcalins (notamment NaCl), les sels alcalino-terreux (notamment $CaCl_2$), les polyols (notamment sorbitol, glycérol, penta-érythrol et polyalkylèneglycols) et leurs mélanges. Ils seront ajoutés sous forme solide ou liquide ou encore sous forme de suspension. De façon pratique, le déclencheur chimique peut être ajouté en solution (notamment à 1 % en poids) dans l'eau de lavage du gâteau humide de séparation. La nature et la dose du déclencheur chimique seront ajustées selon l'effet désiré.

Le déclenchement de la réaction de l'étape (γ) peut être initié, le cas échéant, pendant la séparation de l'étape (β) ou, mieux, après ladite étape (β).

Lors de la mise en oeuvre de l'étape (γ), la siccité augmente progressivement pour atteindre un palier. L'hydratation de la chaux vive dégage de la chaleur (1 160 kJ par kg de CaO à 20°C). Les élévations de température associées à ce dégagement sont d'autant plus importantes que l'on minimise les déperditions calorifiques. On peut, par exemple, contrôler le procédé de l'invention de façon à initier la réaction de l'étape (γ), lorsque la suspension que l'on traite est stockée dans un endroit isolé thermiquement. Les températures requises pour une hygiénisation thermique (i. e. une température supérieure à 55°C pendant 75 minutes) pourront être atteintes sans traitement supplémentaire.

Selon la nature des solides de la suspension à traiter, il est possible d'omettre l'étape (β) de séparation. On obtient alors (voir Ex. 8 et Ex. 9 ci-après) une suspension homogène ou un produit solide qui est humide. La suppression de ladite étape (β) convient parfaitement au traitement des suspensions de matières stercorales.

### Intérêt de l'invention

Le traitement d'une boue selon le procédé de l'invention au moyen de chaux, en remplaçant

(a) le lait de chaux ou

(b) la chaux vive en poudre,

utilisés dans les procédés conventionnels de pré-chaulage ou de post-chaulage, par de la CVRR en poudre offre de nombreux avantages, à savoir en particulier :

- l'obtention d'un mélange homogène lors de l'intervention du $Ca(OH)_2$ de la CVRR puis celle du CaO de ladite CVRR,

- l'absence de risque de réaction violente, alors que la chaux vive, antérieurement utilisée, donne une réaction violente,

- un gain de temps et d'énergie pour obtenir un produit final parfaitement homogène,

- l'absence d'unité de préparation spéciale, alors qu'une telle unité est nécessaire dans le cas d'un lait de chaux,

- une précision de dosage, qui est supérieure à celle de la technique de pré-chaulage au moyen d'un lait de chaux.

La comparaison des résultats des exemples 1-5 selon l'invention avec les exemples comparatifs correspondants A1-A5, met en évidence que le matériau issu d'une boue présente un gain de siccité supérieur ou égal à 2% par rapport aux techniques antérieures de pré-chaulage ou de post-chaulage.

D'autres avantages et caractéristiques de l'invention seront mieux compris à la lecture qui va suivre d'exemples de réalisation (Ex. 1-Ex. 7) et d'exemples comparatifs (A1-A5 correspondant respectivement aux exemples 1 à 5, et exemples 8 à 9). Bien entendu, l'ensemble de ces éléments n'est pas limitatif mais est fourni à titre d'illustration.

*Exemple 1*

Une boue biologique d'origine industrielle, présentant une concentration en matières solides de 2,2% en poids sec, est mélangée sous agitation dans la cuve de 1 m$^3$ d'une centrifugeuse (fonctionnant à 20 m$^3$/h) avec une CVRR ayant un rapport pondéral CaO/Ca(OH)$_2$ de 90/10 et une granulométrie telle que 20 $\mu$m $\leq$ d$_{50}$ $\leq$ 200 $\mu$m, à raison de 1 partie en poids sec de CVRR pour 1 partie en poids sec de matières solides, soit ici 22 kg de CVRR pour 1 m$^3$ de boue à traiter. Une solution aqueuse contenant 500 g/L de chlorure ferrique est ajoutée simultanément dans la cuve à raison de 0,8 L de cette solution pour 1 m$^3$ de boue à traiter. Après centrifugation on obtient un premier gâteau humide ayant un TC de 37 %, dans lequel la CVRR est répartie de façon uniforme. On laisse reposer ce premier gâteau humide de sorte que la réaction du CaO de la CVRR avec l'eau dudit premier gâteau se réalise. Après 72 h, on constate que la siccité du gâteau final a augmenté ; elle est de 42 % et reste constante au-delà de 72 h.

*Exemple comparatif A1*

La boue biologique de l'exemple 1 est traitée conventionnellement, dans la cuve de la même centrifugeuse, avec un floculant organique polymère (ici un copolymère acide acrylique/acrylamide), à raison de 15 g en poids sec de floculant pour 1 kg de matières solides en poids sec de ladite boue. On obtient un premier gâteau humide de séparation, qui est recueilli, présente un TC de 18 %. Ce premier gâteau est ensuite mélangé avec de la chaux vive à raison de 1 partie en poids sec de CaO pour 1 partie en poids sec de matières solides de ladite boue initiale. Après mélange, le gâteau final que l'on obtient est déstructuré par l'action de la chaux vive et présente un TC de 34 % qui, mesuré après 72 h, reste constant.

*Exemple 2*

On opère selon le procédé de l'exemple 1 ci-dessus, mais avec la différence que l'on utilise (a) 0,55 partie en poids sec de CVRR (i. e. 0,5 partie en poids de CaO) pour 1 partie en poids sec de matières solides de la boue initiale, et (b) 0,4 L (au lieu de 0,8 L) de solution aqueuse de FeCl$_3$ pour 1 m$^3$ de boue à traiter. Le second gâteau que l'on recueille a un TC de 31%. Après 72h, on constate que le TC a augmenté et est de 34 % et reste constant au-delà de 72 h.

*Exemple comparatif A2*

On opère selon le procédé de l'exemple comparatif A1, mais avec la différence que l'on utilise pour traiter le premier gâteau, qui a un TC de 18 %, une quantité de 0,5 (au lieu de 1) partie en poids de chaux vive pour 1 partie en poids sec de matières solides de la boue. On obtient un gâteau final ayant un TC de 27,5 %, qui mesuré après 72 h, reste constant.

*Exemple 3*

La boue biologique de l'exemple 1 est traitée dans un filtre-presse avec un ajout de 1 kg de CVRR [ayant un rapport pondéral CaO/Ca(OH)$_2$ de 85/15 et une granulométrie telle que 20 $\mu$m $\leq$ d$_{50}$ $\leq$ 200 $\mu$m] à raison de 1 partie en poids sec de CVRR pour 1 partie en poids sec de matières solides, soit ici 22 kg de CVRR pour 1 m$^3$ de boue à traiter. Une solution aqueuse à 500g/L de FeCl$_3$ est ajoutée simultanément à raison de 0,8 L pour 1 m$^3$ de boue à traiter. Le TC du gâteau final obtenu est de 42 %. Après 72 h, on constate que TC a augmenté et est de 48 %.

*Exemple comparatif A3*

On traite la boue biologique de l'exemple 1 de façon conventionnelle dans un filtre-presse en y ajoutant 1 kg de Ca(OH)$_2$ pour 1 kg de matière sèche contenue dans cette boue. Le premier gâteau de filtration présente un TC de 41 % et une structure ne permettant pas l'opération de post-chaulage.

*Exemple 4*

Une boue biologique d'origine urbaine présentant une teneur en matière sèche de 2,4 % en poids est mélangée, sous agitation, dans une cuve de 1 m$^3$ avec la CVRR de l'exemple 1 ajoutée à raison de 0.5 partie en poids pour 1 partie en poids de matière sèche, soit 12 kg pour 1 m$^3$ de boue à traiter. Une solution aqueuse à 500g/L de FeCl$_3$ est ajoutée simultanément à raison de 0,5 L pour 1 m$^3$ de boue à traiter. Le TC du gâteau final obtenu par séparation sur filtre à bande (opérant avec un débit de 6 m$^3$/h) est de 34 %. Après 72 h, on constate que TC a augmenté et est de 37 %.

*Exemple comparatif A4*

La boue de l'exemple 4 est traitée conventionnellement au moyen du même filtre à bande en y ajoutant 5 g de polymère (polyacrylamide) pour 1 kg de matière sèche. Le premier gâteau de filtration, ainsi obtenu, est mélangé avec de la chaux vive à raison de 0,5 kg de CaO pour 1 kg de matière sèche de la boue initiale. Après mélange, le gâteau déstructuré par l'opération présente un TC de 32 % qui reste constant après 72 h.

*Exemple 5*

400 litres de boue biologique d'origine urbaine présentant une teneur en matières sèches de 3,5 % en poids sont mélangés, sous agitation lente, dans une cuve de 500 litres avec une solution de $FeCl_3$ à raison de 8 parties de $FeCl_3$ pour 100 parties de matière sèche. La CVRR de l'exemple 1 est ensuite introduite directement en poudre sous agitation lente à raison de 45 parties de CVRR pour 100 parties de matière sèche. Le mélange, parfaitement homogène, est pompé sans temps d'attente vers un filtre-presse. Le filtrat est immédiatement clair, le temps de filtration est de 65 minutes, la siccité du gâteau de 35 %.

*Exemple comparatif A5*

La boue de l'exemple 5 est traitée conventionnellement par du chlorure ferrique et de la chaux introduite sous forme de lait de chaux éteinte (l'utilisation d'une chaux vive en poudre ne permettrait pas d'obtenir un mélange homogène compatible avec la filtration sur filtre- presse). Le mélange est pompé vers le filtre-presse après un délai de 30 à 40 minutes afin d'obtenir une clarification immédiate. Le temps de filtration est de 100 minutes et la siccité finale de 33%.

*Exemple 6*

Un lisier de porc homogène présentant une concentration en matière sèche de 5,5% en poids est mélangé, sous agitation, dans une cuve de 1 $m^3$ d'une centrifugeuse, opérant à un débit de 4 $m^3$/h, avec la CVRR de l'exemple 3 ajoutée à raison de 10 kg de CVRR pour 1 $m^3$ de boue à traiter. Le lisier centrifugé se présente immédiatement sous la forme d'une poudre divisée légèrement humide. Après 24 heures, cette poudre est parfaitement sèche et facilement manipulable ; son TC est de 39 %.

*Exemple 7*

Déclenchement de la réaction de post-chaulage par voie chimique

La mesure des réactivités des chaux est réalisée selon la méthode normalisée EN459-2. La modification essentielle, mise en oeuvre ici, porte sur le remplacement de l'eau déminéralisée par une boue biologique ayant une teneur en matières sèches solides de 2,7 %. 150 g de chaux (chaux vive CaO ou CVRR selon l'exemple 1) sont plongés brutalement dans 600 g de cette boue à 20°C. Dès l'introduction, la température du mélange résultant sous agitation est mesurée en continu. Les courbes de montée en température sont présentées à la figure 1. L'additif de déclenchement (HCl) est ajouté, à l'instant A, à raison de 1 % en poids par rapport à la masse de CaO mise en oeuvre.
Dans le système température de la boue (en °C) en fonction de la durée de réaction (en secondes) de la figure 1, la courbe 1 concerne la boue additionnée de chaux vive normale, la courbe 3 concerne la boue additionnée de la CVRR, et la courbe 2 montre l'effet de l'additif de déclenchement après son incorporation à l'instant A dans le mélange boue + CVRR.

*Exemple comparatif 8*

La matière première est une suspension aqueuse de matières stercorales. Elle provient essentiellement d'abattoirs industriels de lapins et a été préalablement centrifugée pour écarter les parties solides.
Cette suspension aqueuse est mélangée en continu et sous agitation avec la CVRR de l'exemple 3 à raison de 5 à 8 parties en poids de CVRR pour 100 parties en poids de matières stercorales qu'elle contient. La CVRR est introduite directement dans le mélangeur sous forme de poudre. Le retard de la réaction (1) permet d'obtenir un mélange homogène à viscosité constante et l'effet de désodorisation est immédiat.
Le mélange homogène ainsi obtenu est transféré dans une unité de stockage où la réaction (1) se déclenche et permet de porter ledit mélange à une température comprise entre 40 et 70°C en fonction de la quantité de CVRR ajoutée. L'augmentation de viscosité après la réaction (1) est également fonction de la quantité de CVRR ajoutée : avec jusqu'à 8 parties de CVRR pour 100 parties de matières stercorales, le mélange résultant reste liquide et est traité comme tel dans la filière d'élimination choisie.

*Exemple comparatif 9*

On reproduit le procédé de l'exemple 8, avec la différence que l'on ajoute 15 parties (au lieu de 5 à 8 parties) de CVRR pour 100 parties de matières stercorales. L'augmentation de la quantité utilisée de CVRR a pour conséquence de faire monter, par le déclenchement de la réaction (1) lors du stockage, la température du mélange résultant à plus de 95°C et d'obtenir une structure solide permettant de stocker le mélange au sol avec un angle de talutage de l'ordre de 45°C. Ce type de traitement peut avantageusement être utilisé en fonction de la filière d'élimination choisie.

**Revendications**

1.  Procédé pour le traitement d'une boue au moyen de chaux en vue de concentrer, dessécher et recueillir ses matières solides, ledit procédé comprenant les étapes consistant à :

    ($\alpha$) mettre en contact et sous agitation pendant 2 à 10 minutes de la chaux vive partiellement pré-hydratée à réactivité retardée (CVRR) avec la boue à traiter, à raison d'au moins 1 partie en poids sec de ladite CVRR pour 100 parties en poids sec de matières solides contenues dans ladite boue, ladite CVRR étant constituée de 85 à 90 % en poids de CaO et de 15 à 10 % en poids de Ca(OH)$_2$-, ayant une granulométrie moyenne telle que 20 $\mu$m $\leq$ d50 $\leq$ 200 $\mu$m et étant utilisée (i) sous forme de poudre ou (ii) sous forme de suspension aqueuse à une concentration supérieure ou égale à 10% en poids, le Ca(OH)$_2$ des particules de ladite CVRR servant à la floculation desdites matières solides ;
    ($\beta$) séparer, notamment par filtration, centrifugation ou décantation, la suspension résultante pour obtenir un matériau solide se présentant sous la forme d'un premier gâteau humide, qui est un mélange homogène desdites matières solides concentrées, de ladite CVRR et d'eau; puis,
    ($\gamma$) faire réagir ou laisser réagir le CaO de ladite CVRR qui est contenue dans ledit matériau solide ainsi séparé, avec l'eau dudit matériau solide.

2.  Procédé selon la revendication 1, **caractérisé en ce que** la mise en contact sous agitation à l'étape ($\alpha$) s'effectue pendant une durée de 2 à 3 minutes.

3.  Procédé suivant la revendication 1 ou 2, **caractérisé en ce que** l'étape ($\alpha$) est mise en oeuvre avec une quantité de CVRR, exprimée en équivalents de CaO, de 3 à 100 parties en poids pour 100 parties en poids sec de matières solides contenues dans ladite boue, ladite CVRR intervenant

    (i) sous la forme de la poudre, ou
    (ii) sous la forme de la suspension aqueuse à une concentration supérieure ou égale à 10 % en poids, de préférence à une concentration supérieure ou égale à 50 % en poids, et mieux (en association dans ce cas avec un superplastifiant) à une concentration de 75 à 90 % en poids,
    **en ce que** la séparation de l'étape ($\beta$) est mise en oeuvre au moyen d'une centrifugeuse, d'un filtre-presse ou d'un filtre a bande, et
    **en ce que** la réaction de l'étape ($\gamma$) est déclenchée, pendant ou après l'étape ($\beta$), notamment par initiation par voie thermique, sonique ou chimique.

4.  Procédé suivant l'une quelconque des revendications précédentes, **caractérisé en ce que** la réaction de l'étape ($\gamma$) est initiée

    • par voie thermique, notamment par apport de vapeur sèche ;
    • par voie sonique, notamment par un champ d'ultrasons ; ou
    • par voie chimique, notamment avec une substance choisie parmi l'ensemble constitué par les acides minéraux, les acides organiques, les sels alcalins, les sels alcalino-terreux, les polyols et leurs mélanges.

5.  Procédé suivant l'une quelconque des revendications précédentes, **caractérisé en ce que** l'étape ($\alpha$) est mise en oeuvre avec une association de la CVRR avec au moins un sel métallique, notamment un sel de Fe$^{3+}$ et/ou un sel de Al$^{3+}$, de préférence FeCl$_3$, Al$_2$(SO$_4$)$_3$ et/ou le chlorure basique d'aluminium.

**Patentansprüche**

1. Verfahren zur Behandlung eines Schlamms mit Kalk zur Konzentration, Austrocknung und Sammlung der darin enthaltenen Feststoffe, umfassend die nachfolgenden Schritte :

   (α) in Verbindung bringen des zu behandelnden Schlamms unter Rühren über 2 - 10 min mit teilweise vorhydratisiertem Branntkalk (CVRR) in einem Verhältnis (bezogen auf das Trockengewicht) von mindestens 1 Teil CVRR pro 100 Gew.-Teile der im Schlamm enthaltenen Feststoffe, wobei der CVRR aus 85 - 90 Gew. % CaO und 15 - 10 Gew. % $Ca(OH)_2$ mit einer derartigen mittleren Körnung von 20 $\mu$m $\leq d_{50} \leq$ 200 $\mu$m und in Form eines Pulvers (i) oder in Form einer wässrigen Suspension (ii) in einer Konzentration von größer oder gleich 10 Gew. % verwendet wird, wobei das $Ca(OH)_2$ der Partikel des CVRR zur Flockung der Feststoffe dient;
   (β) Separieren - insbesondere durch Filtration, Zentrifugation oder Dekantieren - der resultierenden Suspension, um einen Festkörper in Form eines ersten feuchten Kuchens zu erhalten, bei dem es sich um eine homogene Mischung aus den konzentrierten Feststoffen, CVRR und Wasser handelt; gefolgt durch
   (y) Umsetzen des CaO des im derart separierten Festkörper enthaltenen CVRR mit dem Wasser des Festkörpers .

2. Verfahren nach Anspruch 1, **dadurch gekennzeichnet, dass** das Kontaktieren unter Rühren im Schritt (α) über 2 - 3 min erfolgt.

3. Verfahren nach Anspruch 1 oder 2, **dadurch gekennzeichnet, dass** der Schritt (α) mit einer in CaO-Äquivalenten ausgedrückter Menge an CVRR von 3- 100 Gew.-Teilen pro 100 Gew.-Teile Feststoffe im Schlamm erfolgt, wobei der CVRR vorliegt

   (i) in Form des Pulvers oder
   (ii) in Form der wässrigen Suspension in einer Konzentration von mindestens 10 Gew. %, vorzugsweise mindestens 50 Gew. % und besonders bevorzugt (in diesem Fall in Verbindung mit einem Betonverflüssiger) in einer Konzentration von 75 - 90 Gew. %;

   dass die Separation im Schritt (β) mittels einer Zentrifuge, Filterpresse oder eines Bandfilters erfolgt, und dass die Reaktion im Schritt (γ) während oder nach dem Schritt (β) thermisch, durch Beschallung oder chemisch ausgelöst wird.

4. Verfahren nach einem der vorstehenden Ansprüche, **dadurch gekennzeichnet, dass** die Reaktion im Schritt (γ) ausgelöst wird

   • mit thermischen Mitteln, insbesondere durch Zugabe von Trockendampf;
   • durch Beschallung, insbesondere mit einem Ultraschallfeld; oder
   • mit chemischen Mitteln, insbesondere mit einer aus der nachfolgenden Gruppe gewählten Substanz: Mineralsäuren, organische Säuren, alkalische Salze, Erdalkalisalze, Polyole und Mischungen davon.

5. Verfahren nach einem der vorstehenden Ansprüche, **dadurch gekennzeichnet, dass** der Schritt (α) durch eine Assoziation von CVRR mit mindestens einem Metallsalz, insbesondere einem $Fe^{3+}$- und/oder einem $Al^{3+}$-Salz, vorzugsweise $FeCl_3$, $Al_2(SO_4)_3$ und/oder Chlorid, ausgeführt wird.

**Claims**

1. A method for treating sludge by means of lime with a view to concentrating, drying and collecting its solid materials, said method comprising the steps of:

   (α) putting into contact, and under stirring for 2 to 10 minutes, partly prehydrated, delayed-reactivity quick lime (DRQL) with the sludge to be treated, in an amount of at least 1 dry weight part of said DRQL for 100 dry weight parts of solid materials contained in said sludge, said DRQL consisting of 85 to 90% by weight of CaO and of 15 to 10% by weight of $Ca(OH)_2$ having an average grain size such that 20 $\mu$m $\leq$ d50 $\leq$ 200 $\mu$m and being used (i) in the form of powder or (ii) in the form of an aqueous suspension at a concentration greater than or equal to 10% by weight, the $Ca(OH)_2$ of the particles of said DRQL being used for flocculating said solid materials;
   (β) separating, notably by filtration, centrifugation or decantation, the resulting suspension in order to obtain a solid material appearing in the form of a first humid cake, which is a homogeneous mixture of said concentrated solid materials, of said DRQL and of water; then

($\gamma$) reacting or leaving to react the CaO of said DRQL which is contained in said thereby separated solid material, with the water of said solid material.

2. The method according to Claim 1, **characterized in that** the contacting under stirring in step ($\alpha$) is carried out for a period of 2 to 3 minutes.

3. The method according to Claim 1 or 2, **characterized in that** step ($\alpha$) is implemented with an amount of DRQL , expressed in CaO equivalents, from 3 to 100 parts by weight for 100 parts by dry weight of solid materials contained in said sludge, said DRQL intervening

i) in the form of the powder, or
ii) in the form of the aqueous suspension at a concentration that is greater than or equal to 10% by weight, preferably at a concentration that is greater than or equal to 50% by weight, and better (associated in this case with a superplasticizer) at a concentration from 75 to 90% by weight,

**in that** the separation of step ($\beta$) is implemented by means of a centrifuge, a filter press or a strip filter; and
**in that** the reaction of step ($\gamma$) is triggered, during or after step ($\beta$), notably through initiation by a thermal, sonic or chemical route.

4. The method according to any of the preceding claims, **characterized in that** the reaction of step ($\gamma$) is initiated

- by a thermal route, notably by providing dry steam;
- by a sonic route, notably with an ultrasonic field; or
- by a chemical route, notably with a substance selected from the group consisting of mineral acids, organic acids, alkaline salts, earth alkaline salts, polyols and mixtures thereof.

5. The method according to any of the preceding claims, **characterized in that** step ($\alpha$) is implemented with an association of the DRQL with at least one metal salt, notably a $Fe^{3+}$ salt and/or an $Al^{3+}$ salt, preferably $FeCl_3$, $Al_2(SO_4)_3$ and/or basic aluminum chloride.

Fig. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9910288 A
- EP 1154958B A
- EP 1154958 B
- WO 0232818 A
- US 4279279 A
- US 5679262 A
- FR 2841895 A
- JP 60054799 A

**Littérature non-brevet citée dans la description**

- *Wat. Sci. Tech.,* 1993, vol. 28 (1), 223-231
- **S. DENNEUX-MUSTIN et al.** *Wat. Res.,* 2001, vol. 35 (12), 3018-3024